# EUROPÄISCHE PATENTSCHRIFT

(11) **EP 3 166 569 B1**
(45) Veröffentlichungstag und Bekanntmachung des Hinweises auf die Patenterteilung: **28.11.2018**
(21) Anmeldenummer: 15736258.3
(22) Anmeldetag: 09.07.2015
(51) Int. Cl.: A61K 6/083

(54) **KOMPOSITE MIT GESTEUERTER NETZWERKSTRUKTUR**
COMPOSITES WITH CONTROLLED NETWORK STRUCTURE
COMPOSITE À STRUCTURE RÉTICULÉE CONTRÔLÉE

(30) Priorität: 11.07.2014 EP 14176729
(43) Veröffentlichungstag der Anmeldung: 17.05.2017
(73) Patentinhaber: Ivoclar Vivadent AG, 9494 Schaan (LI); Technische Universität Wien, 1040 Wien (AT)
(72) Erfinder: MOSZNER, Norbert, 9495 Triesen (LI); FISCHER, Urs-Karl, CH-9320 Arbon (CH); LAMPARTH, Iris, CH-9472 Grabs (CH); BURTSCHER, Peter, A-6830 Rankweil (AT); LISKA, Robert, A-2123 Schleinbach (AT); GORSCHE, Christian, A-1140 Wien (AT); SEIDLER, Konstanze, A-2105 Oberrohrbach (AT); GAUSS, Paul, A-1180 Wien (AT)
(74) Vertreter: Uexküll & Stolberg
(86) Internationale Anmeldenummer: PCT/EP2015/065771
(87) Internationale Veröffentlichungsnummer: WO 2016/005534

(56) Entgegenhaltungen:
- US-A- 2 856 390
- US-A1- 2012 016 052
- LON J. MATHIAS ET AL: "A novel thioether polymer with pendent ester groups from a readily available acrylate derivative", POLYMER BULLETIN, Bd. 27, Nr. 4, 1. Januar 1992 (1992-01-01) , Seiten 395-398, XP055161025, ISSN: 0170-0839, DOI: 10.1007/BF00309695
- A.Johan H Labuschagne ET AL: "New general syntheses for symmetrical and unsymmetrical organic sulphides", Tetrahedron Letters, 1. Januar 1976 (1976-01-01), Seiten 3571-3574, XP055161022, DOI: 10.1016/S0040-4039(00)71361-X Gefunden im Internet: URL:http://www.sciencedirect.com/science/a rticle/pii/S004040390071361X [gefunden am 2015-01-12]
- HARVEY I W ET AL: "Free Radical Addition Reactions of Allylic Sulfones to Alkenes", TETRAHEDRON, ELSEVIER SCIENCE PUBLISHERS, AMSTERDAM, NL, Bd. 53, Nr. 18, 5. Mai 1997 (1997-05-05), Seiten 6493-6508, XP004105645, ISSN: 0040-4020, DOI: 10.1016/S0040-4020(97)00306-2
- A. A. PUDIKOVA ET AL: "Cascade synthesis of new aryl 2-phenylallyl sulfones from [alpha]-methylstyrene and aromatic mono- and bis-sulfonyl chlorides", RUSSIAN JOURNAL OF ORGANIC CHEMISTRY, Bd. 46, Nr. 3, 1. März 2010 (2010-03-01), Seiten 352-354, XP055161727, ISSN: 1070-4280, DOI: 10.1134/S1070428010030085
- EZQUERRA J ET AL: "Efficient synthesis of 4-methylene-L-glutamic acid and its cyclopropyl analogue", TETRAHEDRON ASYMMETRY, PERGAMON PRESS LTD, OXFORD, GB, Bd. 5, Nr. 5, 1. Januar 1994 (1994-01-01), Seiten 921-926, XP002421057, ISSN: 0957-4166, DOI: 10.1016/S0957-4166(00)86244-8

## Beschreibung

Die vorliegende Erfindung betrifft thermisch- und/oder lichthärtende Komposite zur Herstellung von dentalen Zementen und Füllungskompositen für Inlays, Onlays, Kronen, Brücken oder Verblendmaterialien.

Dentale Komposite, die z.B. als Kompositzement oder als direktes Füllungsmaterial, Inlay, Onlay, Krone oder Verblendwerkstoff eingesetzt werden, enthalten eine polymerisierbare organische Matrix und einen oder mehreren Füllstoffe, die meist mit einem polymerisationsfähigen Haftvermittler oberflächenmodifiziert sind. Je nach Art der Füllstoffe, der Monomermatrix und der Anwendung kann der Füllgrad zwischen ca. 50-90 Gew.-% variieren, wobei Zemente im Vergleich zu Füllungskompositen einen geringeren Füllgrad aufweisen.

Die polymerisierbare organische Matrix enthält in der Regel eine Mischung von Monomeren, Initiator-Komponenten, Stabilisatoren und Pigmenten. Als Harze werden meistens Mischungen von Dimethacrylaten eingesetzt Beispiele hierfür sind die hochviskosen Dimethacrylate 2,2-Bis[4-(2-hydroxy-3-methacryloyloxy-propyl)phenyl]propan (Bis-GMA) und 1,6-Bis-[2-methacryloyloxy-ethoxycarbonylamino]-2,4,4-trimethylhexan (UDMA) oder die als Verdünnermonomere genutzten niedrigerviskosen Dimethacrylate, wie z.B. Bismethacryloyloxymethyltricyclo[5.2.1.]decan (TCDMA), Decandiol-1,10-dimethacrylate (D₃MA) und Triethylenglycoldimethacrylat (TEGDMA).

Bei der radikalischen Polymerisation von dentalen Kompositen kommt es aufgrund des Polymerisationsschrumpfes (ΔV_{P}) der eingesetzten Monomere zu einer Volumenkontraktion, was zur sehr nachteiligen Randspaltbildung bei Füllungskompositen führen kann. Bei der Polymerisation von monofunktionellen Methacrylaten, wie z.B. MMA (ΔV_{P} = 21,0 Vol.-%), führt die Polymerisationsschrumpfung nicht zum Aufbau einer Polymerisationsschrumpfungsspannung (PKS), da die Verringerung des Volumens durch einfaches Fliessen der gebildeten Makromoleküle kompensiert werden kann. Im Falle der vernetzenden Polymerisation von multifunktionellen Methacrylaten bildet sich aber am sogenannten Gelpunkt schon innerhalb weniger Sekunden, d.h. schon bei geringem Monomerumsatz, ein 3-dimensionales Polymernetzwerk aus, weshalb der Polymerisationsschrumpf durch viskoses Fliessen nicht kompensiert werden kann und sich im Material mit zunehmendem Monomerumsatz eine erhebliche PKS aufbaut. Dabei ist die Entwicklung der PKS bei Füllungskompositen von zahlreichen Faktoren, u.a. von der Höhe der Volumenkontraktion während der Polymerisation (Härtung bzw. Nachhärtung), den viskoelastischen Eigenschaften (Elastizitätsmodul und Modulaufbau, Glasübergangstemperatur (T_{G}) von Monomer und Polymer, Viskosität und Fliessverhalten), der Polymerisationskinetik der Polymernetzwerkbildung (Harzfunktionalität, Vernetzungsdichte, Anteil an cyclischen Strukturen, Polymerisationsgeschwindigkeit, Temperatur, Monomer- und Doppelbindungsumsatz), der Art der Härtung und der Art der Restauration (Schichtdicke, Kavitätengeometrie) abhängig. Eine besonders hohe PKS wird bei der Lichthärtung beobachtet (vgl. R. R. Braga, R. Y. Ballester, J. L. Ferracane, Dent. Mater. 21 (2005) 962-970; J. W. Stansbury, Dent. Mater. 28 (2012) 13-22).

Es wurden zahlreiche Strategien zur Reduktion der PKS verfolgt. Das betrifft klinische Methoden, wie z.B. die Inkrement-Schicht-Technik, die Verwendung von Kavitätenlacken mit niedrigem E-Modul zur Bildung einer stressabsorbierden Schicht, der Einsatz von speziellen Belichtungsstrategien (soft-start) oder das Vorerwärmen der Komposite zur Verbesserung der Fliesseigenschaften. Der Einsatz von neuen schrumpfungsarmen Monomeren, z.B. von Monomeren mit ringöffnend polymerisierbaren Gruppen, oder die Verwendung von maßgeschneiderten Vernetzern, z.B. mit photo- oder thermolabilen Spacern kann ebenfalls zu Kompositen mit geringer PKS führen.

Ausserdem wurde versucht, die PKS durch den Zusatz von hyperverzweigten Monomeren, Nanogelen oder Nanotubes sowie von Low-Profile-Additiven oder expandierbaren Füllstoffen zu verringern.

Die WO 98/37104 offenbart ein Verfahren zur Kontrolle des Molekulargewichts bei der Herstellung linearer Polymere durch photoinitiierte radikalische Polymerisation von Vinylmonomeren, bei dem der Photoinitiator zusammen mit einem Additions-Fragmentierungs-Kettenübertragungsreagenz eingesetzt wird.

Die US 5,932,675 offenbart ein Verfahren zur Herstellung von Polymeren mit geringem Molekulargewicht durch radikalische Polymerisation. Die Kontrolle des Molekulargewichts erfolgt durch die Zugabe von Kettenübertragungsreagenzien wie z.B. α-(t-Butanthiomethyl)styrol etc.

Gemäß WO 2006/086646 A2 soll sich die PKS in vernetzten Polymeren durch den Einbau von Gruppen in das Polymernetzwerk verringern lassen, die eine reversible Kettenspaltung ermöglichen. Nach der Härtung werden diese Gruppen beispielsweise durch Bestrahlen mit Licht aktiviert. Dies soll eine reversible Spaltung der Polymerketten bewirken, durch die die PKS abgebaut wird. Zum Einbau dieser Gruppen in die Polymerketten werden Reversible Additions-Fragmentierungs-Kettenübertragungs- (RAFT; Reversible addition-fragmentation chain transfer) Mittel wie z.B. Allylsulfide, Dithiocarbamate und Thiocarbonate eingesetzt.

Die US 2012/0295228 A1 offenbart radikalisch polymerisierbare Dentalmaterialien, die ethylenisch ungesättigte Monomere mit Disulfidgruppen enthalten, die als Additions-Fragmentierungs-Materialien wirksam sind und die PKS reduzieren sollen.

Nachteilig ist, dass der Zusatz von übertragungsaktiven Verbindungen meist zu einer deutlichen Reduktion der Polymerisationsgeschwindigkeit führt, vor allem im Falle von reversiblen Systemen auf der Basis von Dithioestern. Ausserdem ist der Einsatz von Mercaptanen aufgrund Ihres Geruches und der von vielen anderen RAFT-Reagenzien aufgrund ihrer Farbe für dentale Anwendungen praktisch ausgeschlossen.

Der Erfindung liegt die Aufgabe zugrunde, polymerisierbare Dentalwerkstoffe bereitzustellen, die sich im Vergleich zum Stand der Technik durch einen verzögerten Gelpunkt und eine verringerte Polymerisationsschrumpfungsspannung (PKS) bei ähnlichen mechanischen Eigenschaften auszeichnen. Zusätzlich sollen die Materialien eine homogenere Netzwerkarchitektur, eine engere und niedrigere Glasübergangstemperatur und eine verbesserte Schlagzähigkeit aufweisen. Sie sollen weiterhin einen für die intraorale Anwendung akzeptablen Geruch und keine Eigenfarbe haben.

Die Aufgabe wird erfindungsgemäss durch Zusammensetzungen gelöst, die mindestens ein radikalisch polymerisierbares Monomer, mindestens einen Initiator für die radikalische Polymerisation und mindestens eine Verbindung der Formel I enthalten:

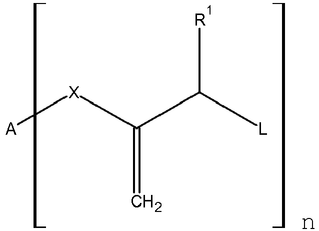

Dabei bedeuten:
- A: ein gesättigter, linearer aliphatischer Kohlenwasserstoffrest mit 1 bis 12 Kohlenstoffatomen, der durch eine oder mehrere 1,4-Phenylengruppen, Urethangruppen oder O unterbrochen sein und der endständig eine polymerisationsfähige Methacryloyloxygruppe tragen kann,
- R¹: H,
- L: -SO₂R³, wobei R³ CH₃ oder Tolyl,
- X: -COO-, -CON(R¹⁰)- oder entfällt, wobei die Bindung an A über O bzw. N erfolgt,
- R¹⁰: Methyl und
- n: 1 oder 2.

Die Formel erstreckt sich nur auf solche Verbindungen, die mit der chemischen Valenzlehre vereinbar sind. Beispielsweise kann n dann, wenn A Wasserstoff ist, nur 1 sein. Der Hinweis, dass ein Rest durch eine oder mehrere 1,4-Phenylengruppen, Urethangruppen, O etc. unterbrochen ist, ist so zu verstehen, dass diese Gruppen in die Kohlenstoffkette des Restes eingeschoben werden. Diese Gruppen sind damit beidseitig durch C-Atome begrenzt und können nicht endständig sein. C₁-Reste können nicht unterbrochen sein.

Die Verbindungen der Formel 1 enthalten mindestens eine radikalisch polymerisierbare Gruppe, wobei Verbindungen mit 2 bis 3 radikalisch polymerisierbaren Gruppen bevorzugt sind.

Polymerisationsübertragungsaktive Verbindungen der Formel I sind teilweise bekannt und lassen sich nach bekannten Synthesemethoden einfach herstellen. So lassen sich Iod-Verbindungen I-L wie unten gezeigt an ein ungesättigtes Derivat addieren, und nachfolgend werden durch HI-Abspaltung erfindungsgemässe Verbindungen der Formel I erhalten:

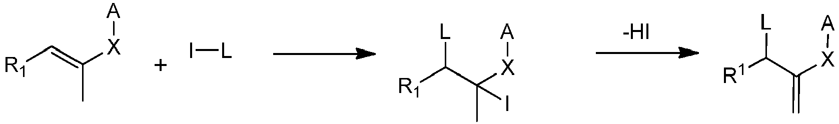

Ein konkretes Beispiel ist:

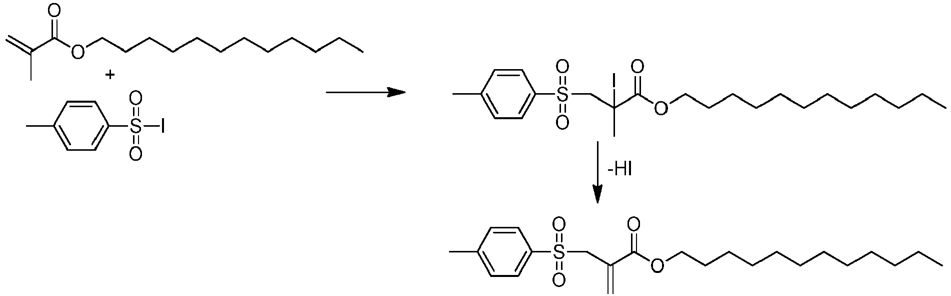

Bevorzugte Beispiele für die erfindungsgemässen polymerisationsübertragungsaktiven Verbindungen der Formel I sind:

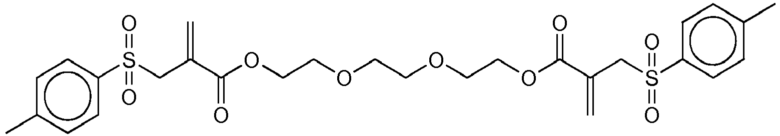

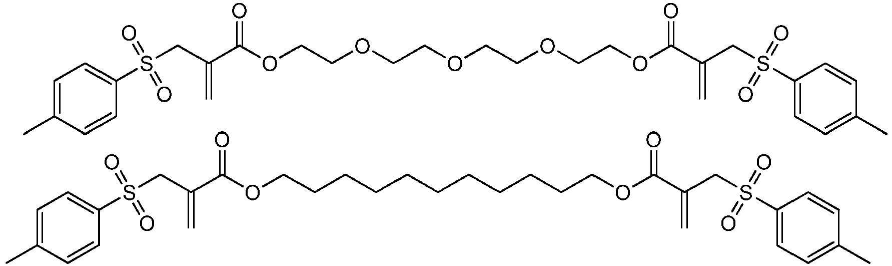

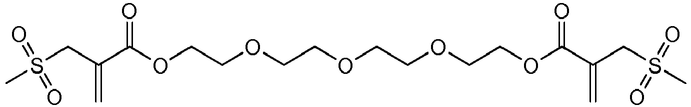

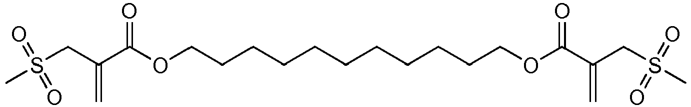

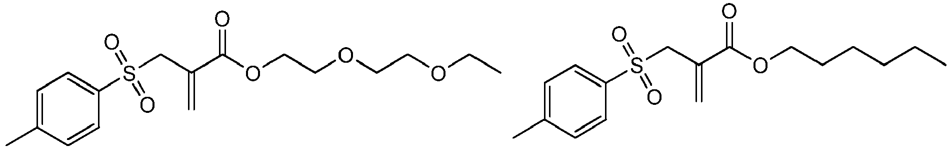

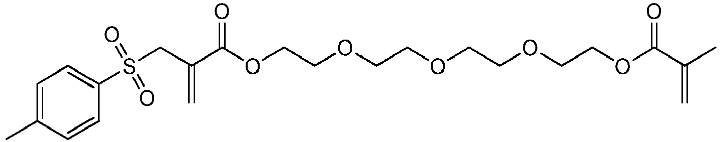

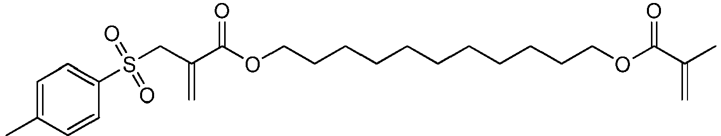

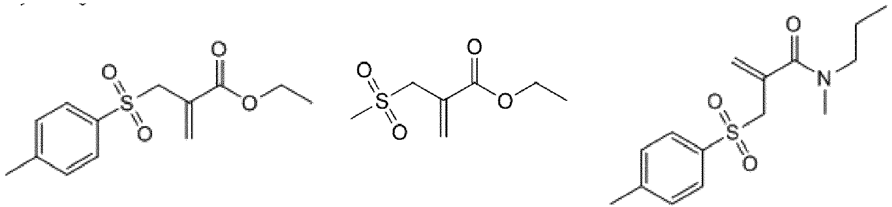

Die Verbindungen der Formel I ermöglichen eine Kontrolle bzw. Steuerung der Netzwerkstruktur bei der radikalischen Polymerisation. Sie führen zu einer deutlich verzögerten Gelbildung und damit zu einer längeren Gelzeit, d.h. dass sich das 3-dimensionale Polymernetzwerk später bildet. Dementsprechend wird eine geringere PKS bei der Aushärtung der Harze bzw. entsprechender Komposite erzielt, was für eine dentale Anwendung z.B. als Füllungsmaterial ein großer Vorteil ist. Die polymerisationsübertragungsaktiven Verbindungen der Formel I ergeben darüber hinaus überraschenderweise auch homogenere Polymernetzwerke mit einem engeren Glasübergang, d.h., dass der Glasübergang in einem engeren Temperaturbereich stattfindet. Dies hat den Vorteil, dass Kettenspannungen durch Relaxationsprozesse besser abgebaut werden und ein schnelleres Debonding-on-Demand (DoD) bewirkt werden kann. Außerdem wird die Glasübergangstemperatur deutlich verringert. Schliesslich ergeben die polymerisationsübertragungsaktiven Verbindungen der Formel I Polymermaterialien mit verbesserter Schlagzähigkeit, was vor allem auf die Verringerung der Glasübergangstemperatur und die homogeneren Netzwerkstruktur zurückzuführen ist. Die verringerte Glasübergangstemperatur hat den weiteren Vorteil, dass die Polymeren bei niedrigeren Temperaturen erweicht werden können. Dies gestattet z.B. im Fall von Adhäsiven und Zementen ein gezieltes Lösen der Klebeverbindung (Debonding-on-Demand).

Neben den Verbindungen der Formel I enthalten die erfindungsgemäßen Zusammensetzungen mindestens ein radikalisch polymerisierbares Monomer, besonders bevorzugt mindestens ein multifunktionelles (Meth)acrylat oder eine Mischung von mono- und multifunktionellen (Meth)acrylaten. Unter monofunktionellen (Meth)acrylaten werden Verbindungen mit einer, unter polyfunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten.

Beispiele für besonders geeignete mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di(meth)acrylat, wie z.B. das Bisphenol-A-dimethacrylat SR-348c (Sartomer) mit 3 Ethoxygruppen oder 2,2-Bis[4-(2-(meth)acryloxypropoxy)phenyl]propan, UD(M)A (ein Additionsprodukt aus 2-Hydroxyethyl(meth)acrylat und 2,2,4-Trimethylhexamethylendiisocyanat), Di-, Tri- oder Tetraethylenglycoldi(meth)acrylat, Trimethylolpropantri(meth)-acrylat, Pentaerythrittetra(meth)acrylat, sowie Glycerindi- und tri(meth)acrylat, 1,4-Butandioldi(meth)acrylat, 1,10-Decandioldi(meth)acrylat (D₃MA) oder 1,12-Dodecandioldi-(meth)acrylat.

Geeignet sind ausserdem auch thermo- oder photolabile Di(meth)acrylate, wie z.B. das Additionsprodukt aus 2 mol 2-Acetoacetoxyethylmethacrylat und 1 mol 2,2,4-Trimethylhexamethylen-1,6-diisocyanat (thermolabil) oder Methacrylsäure-2-[2-(4-{2-methyl-2-[2-(methacryloyloxy)-ethylcarbamoyloxy]propionyl}-phenoxy)-ethoxycarbonylamino]-ethylester. Diese eignen sich besonders zur Herstellung von Werkstoffen mit Debonding-on-Demand-Eigenschaften.

Die erfindungsgemässen dentalen Werkstoffe können neben den oben genannten Co-Monomeren vorzugweise auch radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen. Bevorzugte Monomere mit Carbonsäuregruppen sind Maleinsäure, Acrylsäure, Methacrylsäure, 2-(Hydroxymethyl)-acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure. Bevorzugte Monomere mit Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester. Bevorzugte Monomere mit Phosphorsäuregruppen sind 2-Methacryloyloxypropylmono- oder -dihydrogenphosphat, 2- Methacryloyloxyethylmono- oder -dihydrogenphosphat, 2-Methacryloyloxyethylphenylhydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamidohexyldihydrogenphosphat und 1,3-Bis-(N-acryloyl-N-propylamino)-propan-2-yl-dihydrogenphosphat. Bevorzugte Monomere mit Sulfonsäuregruppen sind Vinylsulfonsäure, 4-Vinylphenylsulfonsäure und 3-(Methacrylamido)propylsulfonsäure.

Zur Initiierung der radikalischen Polymerisation enthalten die erfindungsgemäßen Zusammensetzungen einen Initiator für die radikalische Polymerisation, besonders bevorzugt einen Photoinitiator. Als Photoinitiatoren eignen sich insbesondere Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil. Bevorzugt werden Campherchinon (CQ) und 2,2-Dimethoxy-2-phenyl-acetophenon und besonders bevorzugt α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin, verwendet. Geeignet sind auch Norrish-Typ-I-Photoinitiatoren, vor allem Acyl- oder Bisacylphosphinoxide, und besonders geeignet Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium (MBDEGe). Dabei lassen sich auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Gemäß einer bevorzugten Ausführungsform enthalten die erfindungsgemäßen Dentalwerkstoffe zusätzlich organischen oder vorzugsweise anorganischen partikulären Füllstoff, besonders bevorzugt einen oder mehrere anorganische partikuläre Füllstoffe.

Besonders geeignet sind Füllstoffe auf der Basis von Oxiden mit einer Partikelgröße von 0,01 bis 15 µm, wie SiO₂, ZrO₂ und TiO₂ bzw. Mischoxiden aus SiO₂, ZrO₂, ZnO und/oder TiO₂, nanopartikuläre oder mikrofeine Füllstoffe mit einer Partikelgröße von 10 bis 300 nm, wie pyrogene Kieselsäure oder Fällungskieselsäure sowie Glaspulver mit einer Partikelgröße von 0,01 bis 15 µm, vorzugweise von 0,2 bis 1,5 µm, wie Quarz-, Glaskeramik- oder röntgenopake Glaspulver von z.B. Barium- oder Strontiumaluminiumsilikatgläsern, und röntgenopake Füllstoffe mit einer Partikelgröße von 0,2 bis 5 µm, wie Ytterbiumtrifluorid, Tantal(V)-oxid, Bariumsulfat oder Mischoxide von SiO₂ mit Ytterbium(III)-oxid oder Tantal(V)-oxid. Auch faserförmige Füllstoffe, Nanofasern oder Whiskers sind nicht ausgeschlossen. Wenn nicht anders angegeben handelt es sich bei allen Partikelgrößen um gewichtsmittlere Partikelgrößen.

Die Füllstoffe werden nach der Partikelgröße unterteilt in Makrofüller und Mikrofüller. Makrofüller werden durch Mahlen von Quarz, röntgenopaken Gläsern, Borosilikaten oder von Keramik gewonnen, sind rein anorganischer Natur und bestehen meist aus splitterförmigen Teilen. Bevorzugt sind Makrofüller mit einer mittleren Partikelgröße von 0,2 bis 10 µm. Als Mikrofüller werden vorzugsweise pyrogenes SiO₂ oder Fällungskieselsäure eingesetzt, oder auch Mischoxide, z.B. SiO₂-ZrO₂, die durch hydrolytische Cokondensation von Metallalkoxiden zugänglich sind. Die Mikrofüller haben vorzugsweise eine mittlere Partikelgröße von ca. 5 bis 100 nm.

Zur Verbesserung des Verbundes zwischen den Füllstoffpartikeln und der vernetzten Polymerisationsmatrix können SiO₂-basierende Füllstoffe mit (Meth)acrylat-funktionalisierten Silanen oberflächenmodifiziert werden. Ein Beispiel für solche Silane ist 3-(Meth)acryloyloxypropyltrimethoxysilan. Zur Oberflächenmodifizierung von nichtsilikatischen Füllstoffen wie z.B. von ZrO₂ oder TiO₂ können auch funktionalisierte saure Phosphate, wie z.B. 10-(Meth)acryloyloxydecyldihydrogenphosphat eingesetzt werden.

Der Füllgrad richtet sich nach dem gewünschten Anwendungszweck. Füllungskomposite haben vorzugsweise einen Füllstoffgehalt von 75-90 Gew.-% und Kompositzemente von 50-75 Gew.-%.

Bevorzugte Dentalwerkstoffe enthalten damit neben mindestens einer Verbindung der Formel (I) zusätzlich mindestens ein radikalisch polymerisierbares Monomer, insbesondere mindestens ein multifunktionelles (Meth)acrylat oder eine Mischungen von mono- und multifunktionellen (Meth)acrylaten, mindestens einen Initiator für die radikalische Polymerisation und vorzugsweise auch mindestens einen Füllstoff.

Gegebenenfalls können die erfindungsgemäß eingesetzten Zusammensetzungen weitere Additive enthalten, vor allem Stabilisatoren, Farbmittel, mikrobiozide Wirkstoffe, fluoridionenabgebende Additive, *gasfreisetzende Additive,* optische Aufheller, Weichmacher oder UV-Absorber.

Die erfindungsgemäßen Dentalmaterialien enthalten vorzugsweise 0,5 bis 60 Gew.-%, bevorzugt 1,0 bis 50 Gew.-% und besonders bevorzugt 1,0 bis 40 Gew.-% mindestens einer Verbindung der allgemeinen Formel I.

Zusätzlich enthalten die Materialien vorzugsweise auch 0,01 bis 5,0 Gew.-%, bevorzugt 0,1 bis 5,0 Gew.-% und besonders bevorzugt 0,1 bis 3,0 Gew.-% Initiator(en) für die radikalische Polymerisation, besonders bevorzugt einen Photoinitiator, und besonders bevorzugt auch 5 bis 80 Gew.-%, bevorzugt 10 bis 70 Gew.-% und besonders bevorzugt 10 bis 60 Gew.-% multifunktionelle (Meth)acrylat(e).

Weiterhin enthalten die erfindungsgemäßen Dentalwerkstoffe vorzugsweise 0 bis 90 Gew.-%, bevorzugt 5 bis 90 Gew.-% und besonders bevorzugt 5 bis 80 Gew.-% Füllstoff(e), wobei der Füllstoffgehalt wie oben beschrieben auf die geplante Verwendung der Werkstoffe abgestimmt wird.

Außerdem enthalten die erfindungsgemäßen Dentalwerkstoffe vorzugsweise 0 bis 5 Gew.-%, bevorzugt 0 bis 3 Gew.-%, besonders bevorzugt 0,2 bis 3 Gew.-% sonstige Additiv(e).

Dabei sind erfindungsgemäß Dentalmaterialien besonders bevorzugt, welche die folgenden Komponenten enthalten:
(a) 5 bis 80 Gew.-%, bevorzugt 10 bis 70 Gew.-% und besonders bevorzugt 10 bis 60 Gew.-% multifunktionelle (Meth)acrylat(e),
(b) 0,01 bis 5,0 Gew.-%, bevorzugt 0,1 bis 5,0 Gew.-% und besonders bevorzugt 0,1 bis 3,0 Gew.-% Initiator(en),
(c) 0,5 bis 60 Gew.-%, bevorzugt 1,0 bis 50 Gew.-% und besonders bevorzugt 1,0 bis 40 Gew.-% mindestens einer Verbindung der allgemeinen Formel I,
(d) 0 bis 50 Gew.-%, bevorzugt 0 bis 40 Gew.-% und besonders bevorzugt 0 bis 30 Gew.-% monofunktionelle (Meth)acrylat(e),
(e) 0 bis 90 Gew.-%, bevorzugt 5 bis 90 Gew.-% und besonders bevorzugt 5 bis 80 Gew.-% Füllstoff(e), und
(f) 0 bis 5 Gew.-%, bevorzugt 0 bis 3 Gew.-% und besonders bevorzugt 0,2 bis 3 Gew.-% Additiv(e).

Wenn nicht anders angegeben beziehen sich alle Mengenangaben auf die Gesamtmasse der Materialien. Die einzelnen Mengenbereiche können separat gewählt werden.

Besonders bevorzugt sind solche Dentalwerkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind. Besonders bevorzugt sind außerdem Werkstoffe, die ggf. neben der Verbindung der Formel (I) keine flüchtigen Mercaptane enthalten, d.h. Mercaptane, die einen typischen Mercaptangeruch aufweisen. Ganz besonders bevorzugt sind Zusammensetzungen, die keine weiteren Mercaptane und vorzugsweise auch keine anderen Schwefelverbindungen enthalten.

Die erfindungsgemäßen Dentalwerkstoffe eignen sich besonders als dentale Zemente, Füllungskomposite und Verblendmaterialien sowie als Materialien zur Herstellung von Inlays, Onlays, Kronen und Brücken. Sie haben ähnliche mechanische Eigenschaften (Biegefestigkeit und E-Modul) wie auf Dimethacrylaten basierende Materialien, zeichnen sich jedoch durch eine verringerte Polymerisationsschrumpfungsspannung (PKS), eine verbesserte Schlagzähigkeit und geringen Eigengeruch aus.

Die Dentalwerkstoffe eignen sich primär zur intraoralen Anwendung durch den Zahnarzt zur Restauration geschädigter Zähne (klinische Materialien), z.B. als dentale Zemente, Füllungskomposite und Verblendmaterialien. Sie können aber auch extraoral eingesetzt werden, beispielsweise bei der Herstellung oder Reparatur von Dentalrestaurationen, wie Inlays, Onlays, Kronen und Brücken (technische Materialien).

Die Erfindung wird im Folgenden anhand von Ausführungsbeispielen näher erläutert.

### Ausführungsbeispiele

### Beispiel 1:

### Synthese von 2-(Toluol-4-sulfonylmethyl)-acrylsäurelaurylester (1)

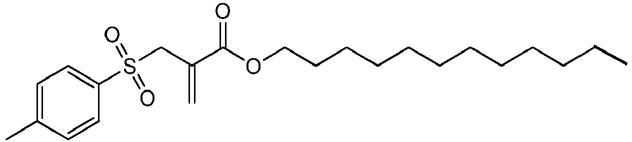

Zunächst wurden im Gelblichtlabor 3,81 g (15 mmol) Iod in 70 ml Ethanol gelöst und langsam in eine 0,1M Lösung von Natrium-p-toluolsulfinat (15 mmol) in Wasser getropft. Der dabei entstehende gelbe Feststoff (4-Methylbenzen-1-sulfonyliodid, MBSI) wurde filtriert und mit Wasser nachgewaschen. Anschließend wurde der Feststoff in CH₂Cl₂ (50 ml) aufgelöst und mit wasserfreiem Na₂SO₄ getrocknet. Das Trockenmittel wurde filtriert, und die Lösung mit dem frisch hergestellten MBSI wurde zusammen mit 2,54 g (10 mmol) Laurylmethacrylat (LMA) gerührt. Die Reaktion wurde mittels Dünnschichtchromatographie (PE/EE 20/1) verfolgt. Nachdem das gesamte LMA aufgebraucht war, wurde die Reaktionslösung mit 5 Gew.-% Natriumdithionit-Lösung (2 x 25 ml) und mit Wasser (1 x 25 ml) gewaschen. Die wässrige Phase wurde mit CH₂Cl₂ rückextrahiert (1 x 25 ml) und die vereinigten organischen Phasen über wasserfreiem Na₂SO₄ getrocknet. Im Anschluss wurden 50 ml Ethylacetat zur Lösung zugegeben und das CH₂Cl₂ wurde am Rotationsverdampfer verdampft. Zur Reaktionslösung wurden nun unter Ar-Atmosphäre 5,06 g (50 mmol) Triethylamin zugegeben und dann über Nacht am Rückfluss gekocht. Nach vollständiger Reaktion wurde die Lösung mit 1N HCl (2 x 50 ml) und dest. Wasser (1 x 50 ml) gewaschen. Die wässrigen Phasen wurden rückextrahiert und die vereinigten organischen Phasen über wasserfreiem Na₂SO₄ getrocknet. Das Lösungsmittel wurde am Rotationsverdampfer abgezogen und das Rohprodukt mit einem Gemisch aus PE/EE 5/1 säulenchromatographisch gereinigt (R_{f} = 0,39). Die Ausbeute betrug ca. 28,8 g (73% der Theorie).

¹H-NMR (200 MHz, CDCl₃, δ): 7.71 (d, *J* = 8.2 Hz, 2H; Ar-H), 7.30 (d, *J* = 8.2 Hz, 2H; Ar-H), 6.47 (s, 1H; =CH₂), 5.89 (s, 1H; =CH₂), 4.12 (s, 2H; -SO₂-CH₂-), 3.94 (t, 2H; -O-CH₂-CH₂-), 2.42 (s, 3H; Ar-CH₃), 1.52 (m, 2H; -O-CH₂-CH₂-), 1.25 (s, 18H; -O-CH₂-CH₂-CH₂-CH₂-CH₂-CH₂-CH₂-CH₂-CH₂-CH₂-CH₂-CH₃) ; 0.86 (m, 3H; -CH₂-CH₃) ;
¹³C-NMR (50 MHz, CDCl₃, δ): 164.9 (C=0), 144.8 (C4), 135.5 (C4), 133.1 (C2), 129.6 (C3), 129.2 (C4), 128.8 (C3), 65.6 (C2), 57.5 (C2), 31.9 (C2), 29.6 (C2, C2, C2), 29.5 (C2), 29.3 (C2), 29.2 (C2), 28.4 (C2), 25.8 (C2), 22.7 (C2), 21.6 (C1), 14.1 (C1).

### Beispiel 2:

### Synthese von Tetraethylenglykol-bis[2-(toluol-4-sulfonylmethyl)acrylat] (2)

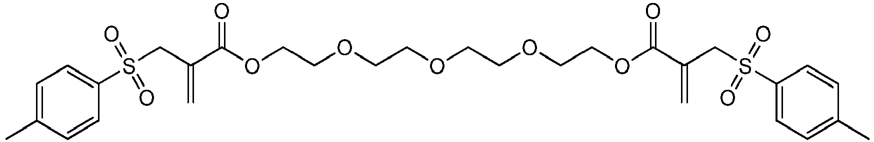

Tetraethylenglycoldimethacrylat (TTEGDMA: 5,29 g, 16 mmol) und 4-Toluolsulfonyliodid (9,03 g, 32 mmol) wurden gemeinsam in CH₂Cl₂ (ca. 50 ml) bei Raumtemperatur im Gelblicht gerührt. Die Reaktion wurde mittels ¹H-NMR-Spektoskopie verfolgt. Nachdem das gesamte TTEGDMA aufgebraucht war (Abnahme der Doppelbindungssignale) wurde die Reaktionslösung mit 5 Gew.-% Natriumdithionit-Lösung (2 x 25ml) und mit Wasser (1 x 25ml) gewaschen. Die wässrige Phase wurde mit CH₂Cl₂ rückextrahiert (1 x 25ml) und die vereinigten organischen Phasen über Na₂SO₄ getrocknet. Im Anschluss wurden 50 ml Ethylacetat zur Lösung zugegeben und das CH₂Cl₂ am Rotationsverdampfer verdampft. Zur Reaktionslösung wurden dann weitere 50 ml Ethylacetat zugegeben und unter Ar-Atmosphäre Triethylamin (8,1 g, 80 mmol) zugetropft (Feststoff fiel aus). Danach wurde die Reaktionslösung über Nacht auf Rückfluss gekocht. Nach vollständiger Reaktion wurde die Lösung mit 1N HCl (2 x 50 ml) und dest. Wasser (1 x 50 ml) gewaschen. Die wässrigen Phasen wurden rückextrahiert und die vereinigten organischen Phasen über Na₂SO₄ getrocknet. Das Lösungsmittel wurde am Rotationsverdampfer abgezogen und das Rohprodukt mit einem Gemisch aus PE/EE 1/4 säulenchromatographisch gereinigt. (R_{f} ∼ 0,32). Ausbeute 70%.

¹H-NMR (200 MHz, CDCl₃, δ): 7.73 (d, *J* = 8.2 Hz, 4H; Ar-H), 7.32 (d, *J* = 8.2 Hz, 4H; Ar-H), 6.52 (s, 2H; =CH₂), 5.89 (s, 2H; =CH₂), 4.14 (m, 8H; OOC-CH₂-, SO₂-CH₂-), 3.62 (m, 12H; -CH₂-O-CH₂-CH₂-), 2.43 (s, 6H; Ar-CH₃).

¹³C-NMR (50 MHz, CDCl₃, δ): 164.9 (C=O), 144.9 (C4), 135.4 (C4), 133.6 (C2), 129.7 (C3), 128.9 (C4), 128.8 (C4), 70.7 (C2), 68.8 (C2), 64.5 (C2), 57.5 (C2), 21.6 (C1).

### Beispiel 3:

### Synthese von Triethylenglykol-bis[2-(toluol-4-sulfonylmethyl)-acrylat] (3)

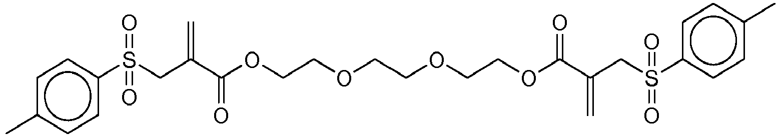

In einem Braunglaskolben wurden nach Beispiel 1 zur Herstellung von MBSI 1 Natrium-p-toluolsulfinat (39,20 g, 0,22 mol) mit Iod (55,83 g, 0,22 mol) umgesetzt und aufgearbeitet. Der gelbe Feststoff wurde in Dichlormethan (300 ml) gelöst. Triethylenglykoldimethacrylat (28,63 g, 0,10 mol) wurde zugegeben und das Reaktionsgemisch wurde bei RT gerührt. Nach 24 h wurde Triethylamin (22,26 g, 0,22 mol) zugetropft. Die rotbraune Lösung wurde 2 h bei Umgebungstemperatur gerührt und anschliessend am Rotationsverdampfer eingeengt. Das dunkelbraune Öl wurde in n-Hexan/Ethylacetat 1:1 (100 ml) aufgenommen und über eine mit Kieselgel gefüllte Fritte filtriert (Kieselgel 60, n-Hexan/Ethylacetat 1:1). Das Filtrat wurde am Rotationsverdampfer eingeengt. Der Rückstand wurde in Ethylacetat (400 ml) gelöst und mit Triethylamin (22,26 g, 0,22 mol) versetzt. Die bräunliche Lösung wurde 6 h am Rückfluss erhitzt. Nach dem Abkühlen wurde die Reaktionslösung mit Salzsäure (1N; 2x 200 ml) und Wasser (200 ml) gewaschen, über Na₂SO₄ getrocknet, filtriert und am Rotationsverdampfer eingeengt. Das bräunliche Öl wurde mittels Säulenchromatographie gereinigt (Kieselgel 60, n-Hexan/Ethylacetat 1:2; R_{f} = 0.35). Man erhielt 48.44 g (81% Ausbeute) eines gelben Öls.

¹H-NMR (200 MHz, CDCl₃, δ): 7.73 (d, *J* = 8.2 Hz, 4H; Ar-H), 7.32 (d, *J* = 8.2 Hz, 4H; Ar-H), 6.52 (s, 2H; =CH₂), 5.89 (s, 2H; =CH₂), 4.14 (m, 8H; OOC-CH₂-, SO₂-CH₂-), 3.62 (m, 12H; -CH₂-O-CH₂-CH₂-), 2.43 (s, 6H; Ar-CH₃) ;
¹³C-NMR (50 MHz, CDCl₃, δ): 164.9 (C=O), 144.9 (C4), 135.4 (C4), 133.6 (C2), 129.7 (C3), 128.9 (C4), 128.8 (C4), 70.7 (C2), 68.8 (C2), 64.5 (C2), 57.5 (C2), 21.6 (C1);

### Beispiel 4:

### Herstellung von Kompositen mit dem Übertragungsreagenz 1 aus Beispiel 1

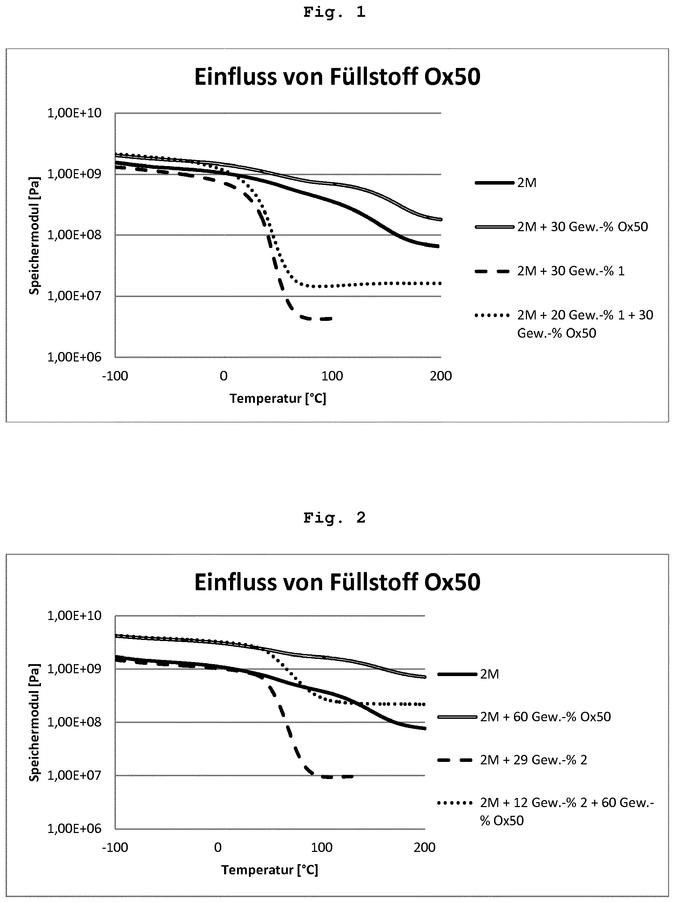

Es wurde eine 1/1-Mischung (mol/mol) der Monomere UDMA und D₃MA hergestellt (Harzmischung 2M). Ein Teil dieser Mischung wurde mit dem Monomer 1 aus Beispiel 1 gemischt. Die zweite Mischung wies die folgende Zusammensetzung auf: UDMA (39 Gew.-%), D₃MA (26 Gew.-%) und 1 (35 Gew.-%). Beide Mischungen wurden mit dem Photoinitiator Ivocerin® (1 Gew.-%) versetzt. Kompositpasten auf der Basis dieser Mischungen wurden durch Zugabe von 30 Gew.-% der pyrogenen Kieselsäure Ox50 hergestellt. Die Kompositpaste mit dem Monomer 1 hatte die Gesamtzusammensetzung: UDMA (27 Gew.-%), D₃MA (18 Gew.-%), 1 (24,3 Gew.-%), Initiator (0,7 Gew.-%) und Ox50 (30 Gew.-%). Die Formulierungen wurden in Silikonformen gegossen und in einem Lumamat 100 (Ivoclar AG) mit dem Programm 2 (P2: 10 min Bestrahlung mit einer Intensität von ca. 20 mW/cm²) polymerisiert. Die Stäbe wurden gewendet und abermals mit P2 ausgehärtet. Die Probestäbchen wurden geschliffen und dann auf einem AntonPaar Rheometer MCR301 mit einem CTD-Ofen (Convection Temperature Control) und einer eingesetzten Festkörpereinspannvorrichtung (SRF12 für rechteckige Querschnitte bis 12 mm) vermessen. Die eingestellte Heizrate betrug 2 °C/min. Alle Proben wurden von -100 °C auf 200 °C aufgeheizt und mit einer konstanten Frequenz von 1 Hz und 0,1% Auslenkung oszilliert. Die in Fig. 1 dargestellten Speichermodulkurven zeigen, dass die Zugabe des Übertragungsreagenz 1 sowohl bei der ausgehärteten Harzprobe als auch im Falle des Komposits gleichermassen zu einer Verringerung der Glasübergangstemperatur und zu einem tieferen und deutlich schmaleren Glasübergangsbereich führt.

| **Formulierung** | **T_{G} [°C]** |
|---|---|
| 2M^{a)}* | 148 |
| 2M + Ox50* | 162 |
| 2M + Monomer 1 | 50 |
| 2M + Monomer 1 + Ox50 | 50 |

| | |
|---|---|
| ^{a)} 2M: UDMA/D₃MA (1/1) * Vergleichsbeispiel | |

### Beispiel 5:

### Herstellung von Kompositen mit dem Übertragungsreagenz 2 aus Beispiel 2

Analog zu Beispiel 4 wurden Prüfkörper hergestellt und untersucht. Dabei wurden folgende Formulierungen verwendet: 1/1-Mischung (mol/mol) von UDMA und D₃MA (Mischung 2M) und eine Mischung von UDMA (43 Gew.-%), D₃MA (28 Gew.-%) mit Monomer 2 (29 Gew.-%). Entsprechende Kompositpasten wurden durch Zugabe von 60 Gew.-% Ox-50 erhalten. Die Kompositpaste mit dem Monomer 2 hatte die folgende Gesamtzusammensetzung: UDMA (17 Gew.-%), D₃MA (11 Gew.-%), 2 (12 Gew.-%) und Ox50 (60 Gew.-%). Fig. 2 zeigt wiederum den Speichermodul in Abhängigkeit von der Temperatur für die ungefüllten und gefüllten Harze. Die in Fig. 2 gezeigten Speichermodulkurven zeigen, dass die Zugabe des Übertragungsreagenz 2 (Beispiel 2) sowohl bei dem ungefüllten Harz als auch im Falle des Komposits zu einem deutlichen schmaleren Glasübergangsbereich mit verringerter Glasübergangstemperatur (T_{G}) führt.

| **Formulierung** | **T_{g} [°C]** |
|---|---|
| 2M^{a)}* | 148 |
| 2M + Ox50* | 157 |
| 2M + Monomer 2 | 73 |
| 2M + monomer 2 + Ox50 | 78 |

| | |
|---|---|
| ^{a)} 2M: UDMA/D₃MA (1/1) * Vergleichsbeispiel | |

### Beispiel 6:

### Herstellung von Füllungskompositen mit dem Übertragungsreagenz 3 aus Beispiel 3

In einem Kneter der Firma Linden wurden die Komposite hergestellt. Dazu wurden zunächst zwei Monomermischungen hergestellt: **Monomermischung A** (alle Angaben in Masse-%): Bis-GMA (28,9%), UDMA (26,0%), SR-348c (14,1%), Kettenübertragungsreagenz 3 (30,0%), Photoinitiator (CQ, 0,2%), EDMAB (0,4%), MBDEGe (0,05%), Lucerin TPO (2,4,6-Trimethylbenzoyldiphenylphosphinoxid; 0,25%), Stabilisator (Hydrochinonmonomethylether (MEHQ), 0,1%). **Monomermischung B:** Bis-GMA (41,3%), UDMA (37,4%), SR-348c (20,3%), CQ (0,2%), EDMAB (0,4%), MBDEGe (0,05%), Lucerin TPO (0,25%), MEHQ (0,1%). Zur Herstellung von Kompositen wurden 22,5 Masse-% der Monomermischung A (Komposit A) bzw. der Monomermischung B (Komposit B) jeweils mit 77,5 Masse-% einer **Füllstoffmischung** eingearbeitet (17% Tetric Evo-Ceram Isofüller (Ivoclar Vivadent AG), 45,5% silanisiertem Ba-Al-Borosilikatglasfüller (mittlere Partikelgröße von 0,7 µm, Schott), 10% Sphärosil (silanisiertes SiO₂-ZrO₂-Mischoxid, mittlere Partikelgrösse von 1,2 µm, Tokoyama Soda), 5% YbF₃ (Ytterbiumtrifluorid, mittlere Partikelgröße von 0,2 µm, Auer Remy; die Prozentangaben beziehen sich jeweils auf die Gesamtmasse des Komposits). Von den Materialien wurden Biegeprüfstäbchen mit einer Länge von 20 mm und einem Querschnitt von 2 x 2 mm präpariert, die 2 mal 3 Minuten mit einer dentalen Lichtquelle (Spectramat®, Ivoclar Vivadent AG) bestrahlt und damit ausgehärtet wurden. Nach der ISO-Norm 4049 (Dentistry - Polymer-based filling, restorative and luting materials) erfolgte die Bestimmung der Biegefestigkeit und des Biege-E-Moduls. Zur Bestimmung der Polymerisationsschrumpfungskraft (PF) wurden die Proben einseitig auf einem silanisierten Objektträger fixiert und mittels eines mit dem Haftvermittler Monobond (Ivoclar Vivadent AG) behandelten Stahlstempels (d = 10 mm) mit einer Zwick Universalprüfmaschine verbunden. Nach dem Einstellen der Schichtdicke (0,8 mm) und Entfernen des Überschusses wurde die Messung gestartet.

Die Belichtung (Bluephase 20i, High Power, 10 s) erfolgte durch den Objektträger hindurch und startete 120 s nach Messbeginn. Während insgesamt 10 Minuten wurde die Kraftänderung bei konstant gehaltener Traversenposition registriert. Die PKS wurde durch Division der gemessenen Kraft durch die Fläche der Prüfkörper erhalten. Die resultierenden Messwerte sind in der Tabelle 1 zusammengestellt. Die Ergebnisse belegen, dass die Kompositprobe A mit einer Verbindung der Formel I eine signifikant reduzierte Polymerisationsschrumpfungskraft zeigt und dabei im Vergleich zum Referenzkomposit B keine schlechteren mechanischen Eigenschaften aufweist.

**Tabelle 1: Eigenschaften der Komposite**

| **Eigenschaft** | **Komposit A** | **Komposit B*** |
|---|---|---|
| Biegefestigkeit (MPa) nach 24 h | 105.3±5.9 | 115.0±12.9 |
| Biegefestigkeit (MPa) nach 24 h WL¹⁾ | 140.9±10.3 | 129.1±8.0 |
| Biege-E-Modul (GPa) nach 24 h | 9.57±0.73 | 10.23±0.38 |
| Biege-E-Modul (GPa) nach 24 h WL | 9.91±0.56 | 10.00±0.20 |
| PKS (MPa) nach 80 s | 0,18 | 0,26 |
| PKS (MPa) nach 480 s | 0,23 | 0,30 |

| | | |
|---|---|---|
| ¹⁾ WL = Wasserlagerung der Prüfkörper * Vergleichsbeispiel | | |

### Beispiel 7:

### Messung der Schlagzähigkeit (Dynstat-Impact-Test)

Die Ermittlung der Zähigkeitseigenschaften erfolgte mittels der DYNSTAT-Anordnung entsprechend der DIN 53435, wobei die Schlagzähigkeit (Schlagarbeit) von ungekerbten Prüfkörpern in der Schlagbiegeanordnung ermittelt wurde. Es wurden Probestäbe (∼ 1 x 0,45 x 0,15 cm) aus den in Tabelle 2 genannten Formulierungen hergestellt und Dynstat-Impact Tests unter Verwendung eines 2 kpcm-Hammers (0,2 J) durchgeführt. In der unten angeführter Tabelle 2 sind die erhaltenen Werte aufgelistet.

**Tabelle 2: Schlagzähigkeit**

| **Formulierung** | **Schlagarbeit [kJ/m²]^{a)}** |
|---|---|
| 2M^{b)}* | 4,0 |
| 2M + Monomer 2 (25 Gew.-%) | 6,8 |
| 2M + Monomer 2 (29 Gew.-%) | 18,0 |

| | |
|---|---|
| ^{a)} Normiert auf Breite und Dicke ^{b)} 2M: UDMA/D₃MA (1/1) * Vergleichsbeispiel | |

Es ist klar ersichtlich, dass eine Schlagzähigkeitserhöhung erreicht werden konnte. Die Schlagzähigkeit konnte bei einem Anteil von 25 Gew.-% der Verbindung 2 (Übertragungsreagenz) um mehr als 50% gesteigert werden.

### Beispiel 8:

### Synthese von 2-(Tosylmethyl)acrylsäure (10)

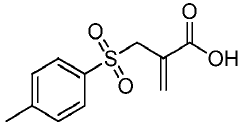

*Brommethacrylsäure* (8,25 g, 50 mmol) wurde in 250 ml heißem MeOH gelöst und mit NaOH (2 g, 50 mmol) versetzt. Anschließend wurde Natrium-p-toluolsulfinat (8,91 g, 50 mmol) portionsweise zugegeben und 2 h auf Rückfluss erhitzt. Nach dem Abziehen des Lösungsmittels an einem Rotationsverdampfer wurde der feste Rückstand in 500 ml Wasser aufgenommen und *2-(Tosylmethyl)-acrylsäure* 10 mit 1 N HCl ausgefällt. Ausbeute 7,44 g (62 %d.Th.) .

¹H NMR (200 MHz, CDCl₃) δ= 8,81 (bs, 1H), 7,67 (d, J= 8,6 Hz, 2 H; Ar-H), 7,27 (d, J= 8,6 Hz, 2 H; Ar-H), 6,55 (s, 1 H; C=H₂), 5,94 (s, 1 H; C=H₂), 4,04 (s, 2 H; -SO₂-CH₂-), 2,36 (s, 3 H; Ar-CH₃) ppm.

### Beispiel 9:

### Synthese von N-Methyl-N-propyl-2-(tosylmethyl)acrylamid (11)

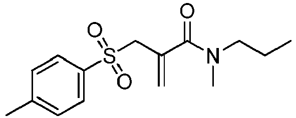

*2-(Tosylmethyl)acrylsäure* 10 (3,00 g, 12,5 mmol) wurde in 30 ml *Thionylchlorid* 2 h lang auf Rückfluss erhitzt. Nach dem Abziehen des überschüssigen SOCl₂ wurde das Säurechlorid in 100 ml Dichlormethan aufgenommen und bei 0 °C langsam mit 6 Äquivalenten *Propylmethylamin* versetzt. Nach 12 h Rühren bei RT wurde das Lösungsmittel am Rotationsverdampfer abgezogen und das Rohprodukt, nach der Aufnahme in 20 ml Dichlormethan, mit 1 N HCl (2 x 20 ml) und gesättigter NaCl Lösung (1 x 20 ml) gewaschen. Nach säulenchromatographischer Reinigung (PE:EE (1:1) + 0,5 %iger Essigsäure) wurden 701 mg (19 %d.Th.) N-*Methyl-N-propyl-2-(tosylmethyl)acrylamid* 11 erhalten.

¹H NMR (200 MHz, CDCl₃) δ= 7,78 (d, J= 8,1 Hz, 2 H; Ar-H), 7,34 (d, J= 8,1 Hz, 2 H; Ar-H), 5,51 (bs, 1 H; C=H₂), 5,41 (s, 1 H; C=H₂), 4,09 (s, 2 H; -SO₂-CH₂-), 3,5-2,7 (m, 5 H; N-CH₃, N-CH₂-CH₂-CH₃), 2,38 (s, 3 H; Ar-CH₃), 1,7-1,3 (m, 2H; N-CH₂-CH₂-CH₃), 0,85 (t, J= 7,5 Hz, 3 H; N-CH₂-CH₂-CH₃) ppm.

### Beispiel 10:

### Synthese von 14-Methyl-13-oxo-3,6,9,12-tetraoxapentadec-14-en-1-yl 2-(tosylmethyl)acrylat (12)

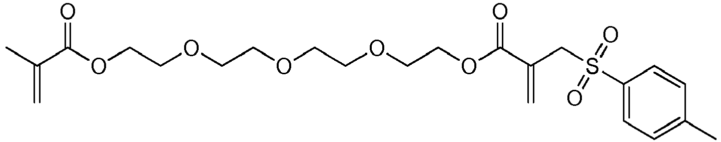

Tetraethylenglykoldimethacrylat (TTEGDMA, 14,6 g, 44,7 mmol) und 4-Toluolsulfonyliodid (12,6 g, 44,7 mmol) wurden gemeinsam in CH₂Cl₂ (ca. 100 ml) bei Raumtemperatur im Gelblicht gerührt. Die Synthese erfolgte anlog der Synthese von Beispiel 2. Es wurde Triethylamin (22,6 g, 223,4 mmol) zugetropft (Feststoff fiel aus). Das Rohprodukt wurde mit einem Gemisch aus PE/EE 1/3 säulenchromatographisch gereinigt. (R_{f} ∼ 0,38). Ausbeute 24%.

¹H-NMR (200 MHz, CDCl₃, δ): 7,70 (d, *J* = 8,2 Hz, 2H; Ar-H), 7,31 (d, *J* = 8,2 Hz, 2H; Ar-H), 6,49 (s, 1H; =CH₂), 6,10 (m, 1H; =CH₂), 5,86 (s, 1H; =CH₂), 5,50 (m, 1H; =CH₂), 4,27 (m, 2H; -OOC-CH₂-), 4,12 (m, 4H; -OOC-CH₂-, -SO₂-CH₂-), 3,71 (m, 2H; OOC-CH₂-CH₂-), 3,63 (m, 10H; -CH₂-O-CH₂-CH₂-O-CH₂-CH₂-), 2,41 (s, 6H; Ar-CH₃) 1,92 (m, 3H; -CO-C-CH₃).

¹³C-NMR (50 MHz, CDCl₃, δ): 164,9 (C=O), 145,0 (C4), 136,2 (C4) 135,5 (C4), 133,7 (C2), 129,8 (C3), 129,0 (C4), 128,9 (C3), 125,9 (C2), 70,7 (C2), 69,2 (C2), 68,9 (C2), 64,6 (C2), 64,0 (C2), 57,7 (C2), 21,8 (C1), 18,4 (C1).

### Beispiel 11:

### Synthese von 2-(Methylsulfonylmethyl)-acrylsäureethyl-ester (13)

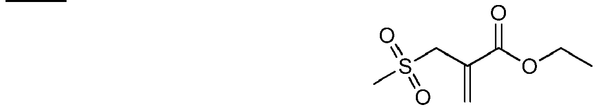

Ethyl-2-(bromomethyl)acrylat (1,1 g, 5,8 mmol), Natriummethansulfinat (0,7 g, 6,7 mmol) und 0,1 g Polyethylenoxid 400 wurden unter Argon-Atmosphäre in 10 mL absolutem THF vorgelegt. Anschließend wurde für 20 Stunden auf Rückfluss erhitzt, wobei der Reaktionsfortschritt mittels NMR und DC überwacht wurde. Nach vollständiger Reaktion wurde die Reaktionslösung mit 10 ml deionisiertem Wasser und 10 ml Diethylether verdünnt. Die wässrige Phase wurde dreimal mit je 25 ml Diethylether extrahiert. Die vereinigten organischen Phasen wurden danach mit gesättigter Kochsalzlösung gewaschen, über Natriumsulfat getrocknet und am Rotationsverdampfer eingedampft. Das erhaltene Rohprodukt wurde säulenchromatographisch mit einem Gemisch aus PE/EE 1/1 gereinigt. (R_{f} ∼ 0,45). Ausbeute 33%.

¹H-NMR (200 MHz, CDCl₃, δ): 6,64 (s, 1H; =CH₂), 6,15 (s, 1H; =CH₂), 4,28 (q, *J* = 7,1 Hz, 2H; -COO-CH₂-CH₃), 4,06 (s, 2H; -SO₂-CH₂-C-), 2,90 (s, 3H; -SO₂-CH₃), 1,33 (t, *J* = 7,1 Hz, 3H; -COO-CH₂-CH₃).
¹³C-NMR (50 MHz, CDCl₃, δ) : 165,3 (C=O), 133,9 (C2), 129,1 (C4), 61,9 (C2), 56,4 (C2), 40,5 (C1), 14,1 (C1).

### Beispiel 12:

### Herstellung und Charakterisierung von Polymeren mit Dimethacrylaten und Übertragungsreagenzien

Es wurde eine 1/1-Mischung (mol/mol) von UDMA und D₃MA (2M) sowie Mischungen von UDMA, D₃MA und jeweils einem Übertragungsreagenz (Verbindungen Nr. 1, 2, 11-13) gemäß Tabelle 3 hergestellt. Die Formulierungen enthielten zusätzlich 1 Gew.-% Ge-Initiator (Ivocerin). Zur Überprüfung der Photoreaktivität wurden die hergestellten Formulierungen mit einem Photorheometer (Modell MCR 302 WESP, Anton Paar) vermessen. Es wurde ein Platte/Platte- Messsystem des Typs PP25 verwendet, und der Messspalt wurde auf 0,1 mm eingestellt. Vor und während der Aushärtung mit einer UV-Lampe (Modell Omniucure 2000; 400-500 nm; 1 W/cm² bzw. 3 W/cm²) wurden Speicher- und Verlustmodul der Probe im Oszillationsmodus (1 % Auslenkung, 1 Hz) gemessen. Als Maß für die auftretende Polymerisationsschrumpfung dient der erreichte Doppelbindungsumsatz (DBC) am Gelpunkt (Schnittpunkt von Speicher- und Verlustmodul). Der Doppelbindungsumsatz bis zum Gelpunkt führt nicht zum Aufbau von Spannungen, da die auftretende Polymerisationsschrumpfung durch Fliessprozesse kompensiert wird. Je höher der Doppelbindungsumsatz bis zum Gelpunkte ist, umso geringer sind folglich der Doppelbindungsumsatz und der Polymerisationsschrumpf im Gelzustand, was somit auch zu einer geringeren Polymerisationsschrumpfkraft führt. Zur Ermittlung des Glasüberganges wurden die Formulierungen in Silikonformen gegossen und in einem Lichtofen (Modell Lumamat 100, Ivoclar AG) mit dem Programm 2 (P2: 10 min Bestrahlung mit einer Intensität von ca. 20 mW/cm²) polymerisiert. Die Stäbe wurden gewendet und abermals mit P2 ausgehärtet. Die Probestäbchen wurden geschliffen und dann auf einem Rheometer (Modell MCR 302) mit einem CTD-Ofen (Convection Temperature Control) und einer eingesetzten Festkörpereinspannvorrichtung (SRF12 für rechteckige Querschnitte bis 12 mm) vermessen. Die eingestellte Heizrate betrug 2 °C/min. Alle Proben wurden von -100 °C auf 200 °C aufgeheizt und mit einer konstanten Frequenz von 1 Hz und 0,1 % Auslenkung oszilliert.

Die in Tabelle 3 gezeigten Glasübergangstemperaturen (Maxima der Verlustmodulkurven) zeigen, dass die Zugabe der Übertragungsreagenzien zu einem tieferen und deutlich schmaleren Glasübergangsbereich führt, was bei den erfindungsgemäßen Zusammensetzungen ein Debonding-on-Demand erheblich erleichtert. Ausserdem ist ersichtlich, dass der Doppelbindungsumsatz am Gelpunkt durch die Übertragungsreagenzien erhöht wird. Damit ist eine geringere Schrumpfungsspannung zu erwarten, weil bis zum Gelpunkt Spannungen durch Fliessprozesse abgebaut werden können.

**Tabelle 3**

| **Formulierung** | **T_{G} [°C]** | **HWB [°C]** | **DBC [%]** |
|---|---|---|---|
| 2M^{a)}* | 150 | 152 | 18 |
| 2M^{c)}* | 148 | 145 | 18 |
| 2M + 35 Gew.-% 1 | 50 | 28 | ^{b)} |
| 2M + 29 Gew.-% 2 | 75 | 32 | 23 |
| 2M + 34 Gew.-% 11^{c)} | 74 | 57 | 36 |
| 2M + 45 Gew.-% 12 | 64 | 23 | ^{b)} |
| 2M + 20 Gew.-% 13 | 66 | 26 | ^{b)} |

| | | | |
|---|---|---|---|
| * Vergleichsbeispiel T_{G} Glasübergangstemperatur HWB Halbwertsbreite DBC Doppelbindungsumsatz am Gelpunkt ^{a)} 2M: UDMA/D₃MA (1/1) ^{b)} nicht gemessen ^{c)} Aushärtung mit 3 W/cm² | | | |

## Patentansprüche

1. Radikalisch polymerisierbarer Dentalwerkstoff, der mindestens ein radikalisch polymerisierbares Monomer, mindestens einen Initiator für die radikalische Polymerisation und mindestens eine Verbindung der Formel I enthält: 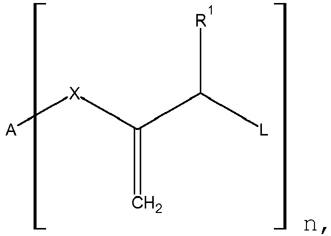 wobei die Variablen der Formel I die folgenden Bedeutungen haben:
A ein gesättigter, linearer aliphatischer Kohlenwasserstoffrest mit 1 bis 12 Kohlenstoffatomen, der durch eine oder mehrere 1,4-Phenylengruppen, Urethangruppen oder O unterbrochen sein und der endständig eine polymerisationsfähige Methacryloyloxygruppe tragen kann,
X -COO- oder -CON(R¹⁰)-, wobei R¹⁰ Methyl ist, oder entfällt, wobei die Bindung an A über O bzw. N erfolgt;
R¹ H,
L -SO₂R³, wobei R³ CH₃ oder Tolyl ist,
n 1 oder 2.

2. Dentalwerkstoff nach Anspruch 1, wobei die Variablen der Formel I die folgenden Bedeutungen haben:
A ein gesättigter, linearer aliphatischer Kohlenwasserstoffrest mit 6 bis 12 Kohlenstoffatomen, der durch 1 bis 3 O-Atome unterbrochen sein kann,
X -COO-;
R¹ H,
L SO₂R³, wobei R³ CH₃ oder Tolyl ist,
n 1 oder 2.

3. Dentalwerkstoff nach einem der Ansprüche 1 bis 2, der mindestens ein multifunktionelles (Meth)acrylat oder eine Mischung von mono- und multifunktionellen (Meth)-acrylaten enthält.

4. Dentalwerkstoff nach einem der Ansprüche 1 bis 3, der mindestens einen Füllstoff enthält.

5. Dentalwerkstoff nach einem der Ansprüche 1 bis 4, der zusätzlich mindestens einen Photoinitiator für die radikalische Polymerisation enthält.

6. Dentalwerkstoff nach einem der Ansprüche 1 bis 5, der
0,5 bis 60 Gew.-%, bevorzugt 1,0 bis 50 Gew.-% und besonders bevorzugt 1,0 bis 40 Gew.-% mindestens einer Verbindung der allgemeinen Formel I,
0,01 bis 5,0 Gew.-%, bevorzugt 0,1 bis 5,0 Gew.-% und besonders bevorzugt 0,1 bis 3,0 Gew.-% Initiator(en) für die radikalische Polymerisation, und ggf.
5 bis 80 Gew.-%, bevorzugt 10 bis 70 Gew.-% und besonders bevorzugt 10 bis 60 Gew.-% multifunktionelle (Meth)acrylat(e) enthält, jeweils bezogen auf die Gesamtmasse des Dentalwerkstoffs.

7. Dentalwerkstoff nach Anspruch 6, der die folgende Zusammensetzung aufweist:
(a) 5 bis 80 Gew.-%, bevorzugt 10 bis 70 Gew.-% und besonders bevorzugt 10 bis 60 Gew.-% multifunktionelle (Meth)acrylat(e),
(b) 0,01 bis 5,0 Gew.-%, bevorzugt 0,1 bis 5,0 Gew.-% und besonders bevorzugt 0,1 bis 3,0 Gew.-% Initiator(en),
(c) 0,5 bis 60 Gew.-%, bevorzugt 1,0 bis 50 Gew.-% und besonders bevorzugt 1,0 bis 40 Gew.-% mindestens einer Verbindung der allgemeinen Formel I,
(d) 0 bis 50 Gew.-%, bevorzugt 0 bis 40 Gew.-% und besonders bevorzugt 0 bis 30 Gew.-% monofunktionelle (Meth)acrylat(e),
(e) 0 bis 90 Gew.-%, bevorzugt 5 bis 90 Gew.-% und besonders bevorzugt 0 bis 80 Gew.-% Füllstoff(e), und
(f) 0 bis 5 Gew.-%, bevorzugt 0 bis 3 Gew.-% und besonders bevorzugt 0,2 bis 3 Gew.-% Additiv(e),
jeweils bezogen auf die Gesamtmasse des Dentalwerkstoffs.

8. Dentalwerkstoff nach einem der Ansprüche 1 bis 7, der keine flüchtigen Mercaptane, bevorzugt überhaupt keine Mercaptane und vorzugsweise auch keine anderen Schwefelverbindungen enthält.

9. Dentalwerkstoff nach einem der Ansprüche 1 bis 8 zur intraoralen Verwendung zur Restauration geschädigter Zähne.

10. Dentalwerkstoff nach Anspruch 9 zur Verwendung als Zement, Füllungskomposit oder Verblendmaterial.

11. Verwendung eines Dentalwerkstoffs nach einem der Ansprüche 1 bis 8 als Material zur extraoralen Herstellung oder Reparatur von Dentalrestaurationen.

12. Verwendung nach Anspruch 11 zur Herstellung von Inlays, Onlays, Kronen oder Brücken.

13. Verwendung einer Verbindung der Formel (I), in der die Variablen wie in Anspruch 1 oder 2 definiert sind, zur Verringerung der Polymerisationsschrumpfungsspannung von Dentalwerkstoffen.

## Claims

1. Radically polymerisable dental material that comprises at least one radically polymerisable monomer, at least one initiator for the radical polymerisation and at least one compound of Formula I: 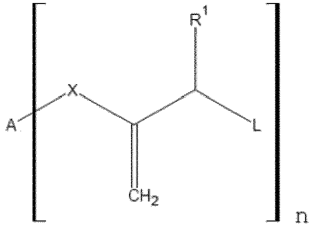 wherein the variables of Formula I have the following meanings:
A = a saturated, linear aliphatic hydrocarbon group having 1 to 12 carbon atoms which may be interrupted by one or more 1,4-phenylene groups, urethane groups or O and which may carry a polymerisable methacryloyloxy group in the terminal position,
X = -COO-, -CON(R¹⁰)-, wherein R¹⁰ is methyl or is absent, wherein the bond to A is through O or N;
R¹ = H,
L = -SO₂R³, wherein R³ is CH₃ or tolyl,
n = 1 or 2.

2. Dental material according to claim 1, wherein the variables of Formula I have the following meanings:
A = a saturated, linear aliphatic hydrocarbon group having 6 to 12 carbon atoms which may be interrupted by 1 to 3 O atoms,
X = -COO-,
R¹ = H,
L = SO₂R³, wherein R³ is CH₃ or tolyl,
n = 1 or 2.

3. Dental material according to one of claims 1 to 2 which comprises at least one multifunctional (meth)acrylate or a mixture of mono- and multifunctional (meth)acrylates.

4. Dental material according to one of claims 1 to 3 which comprises at least one filler.

5. Dental material according to one of claims 1 to 4 which additionally comprises at least one photoinitiator for the radical polymerisation.

6. Dental material according to one of claims 1 to 5 which comprises
0.5 to 60 wt %, preferably 1.0 to 50 wt % and particularly preferably 1.0 to 40 wt % of at least one compound of the general Formula I,
0.01 to 5.0 wt %, preferably 0.1 to 5.0 wt % and particularly preferably 0.1 to 3.0 wt % of initiator(s) for the radical polymerisation, and optionally
5 to 80 wt %, preferably 10 to 70 wt % and particularly preferably 10 to 60 wt % of multifunctional (meth)acrylate(s), in each case relative to the total mass of the dental material.

7. Dental material according to claim 6 which has the following composition:
(a) 5 to 80 wt %, preferably 10 to 70 wt % and particularly preferably 10 to 60 wt % of multifunctional (meth)acrylate(s),
(b) 0.01 to 5.0 wt %, preferably 0.1 to 5.0 wt % and particularly preferably 0.1 to 3.0 wt % of initiator(s),
(c) 0.5 to 60 wt %, preferably 1.0 to 50 wt % and particularly preferably 1.0 to 40 wt % of at least one compound of the general Formula I,
(d) 0 to 50 wt %, preferably 0 to 40 wt % and particularly preferably 0 to 30 wt % of monofunctional (meth)acrylate(s),
(e) 0 to 90 wt %, preferably 5 to 90 wt % and particularly preferably 0 to 80 wt % of filler(s), and
(f) 0 to 5 wt %, preferably 0 to 3 wt % and particularly preferably 0.2 to 3 wt % additive(s),
in each case relative to the total mass of the dental material.

8. Dental material according to one of claims 1 to 7 which does not comprise volatile mercaptans, preferably does not comprise any mercaptans at all and preferably also does not comprise other sulfur compounds.

9. Dental material according to one of claims 1 to 8 for intraoral use to restore damaged teeth.

10. Dental material according to claim 9 for use as cement, filling composite or veneering material.

11. Use of a dental material according to one of claims 1 to 8 as a material for the extraoral manufacture or repair of dental restorations.

12. Use according to claim 11 for the manufacture of inlays, onlays, crowns or bridges.

13. Use of a compound of Formula (I), in which the variables are as defined in claim 1 or 2, to reduce the polymerisation shrinkage stress of dental materials.

## Revendications

1. Matériau dentaire polymérisable par voie radicalaire, qui contient au moins un monomère polymérisable par voie radicalaire, au moins un amorceur de polymérisation par voie radicalaire et au moins un composé de formule I : 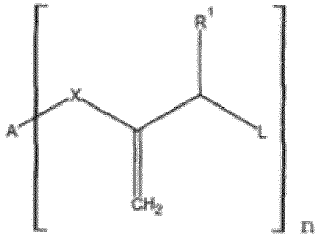 dans laquelle formule I les symboles ont les significations suivantes :
- A représente un reste d'hydrocarbure aliphatique linéaire saturé, comportant de 1 à 12 atomes de carbone, qui peut être interrompu par un ou plusieurs groupe(s) 1,4-phénylène, groupe(s) uréthane ou atome(s) d'oxygène et qui peut porter à son extrémité un groupe méthacryloyl-oxy apte à la polymérisation,
- X représente un fragment symbolisé par -CO-O- ou -CO-N(R¹⁰)-où R¹⁰ représente un groupe méthyle, ou ne représente rien, étant entendu que la liaison au reste A est établie par l'intermédiaire de l'atome d'oxygène ou de l'atome d'azote,
- R¹ représente un atome d'hydrogène,
- L représente un groupe de formule -SO₂R³ où R³ représente un groupe méthyle ou tolyle,
- et l'indice n vaut 1 ou 2.

2. Matériau dentaire conforme à la revendication 1, dans lequel les symboles de la formule I ont les significations suivantes :
- A représente un reste d'hydrocarbure aliphatique linéaire saturé, comportant de 6 à 12 atomes de carbone, qui peut être interrompu par 1 à 3 atome(s) d'oxygène,
- X représente un fragment symbolisé par -CO-O-
- R¹ représente un atome d'hydrogène,
- L représente un groupe de formule -SO₂R³ où R³ représente un groupe méthyle ou tolyle,
- et l'indice n vaut 1 ou 2.

3. Matériau dentaire conforme à l'une des revendications 1 à 2, qui contient au moins un acrylate ou méthacrylate polyfonctionnel ou un mélange d'acrylates et/ou méthacrylates monofonctionnels et poly-fonctionnels.

4. Matériau dentaire conforme à l'une des revendications 1 à 3, qui contient au moins une charge.

5. Matériau dentaire conforme à l'une des revendications 1 à 4, qui contient en outre au moins un photoamorceur de polymérisation par voie radicalaire.

6. Matériau dentaire conforme à l'une des revendications 1 à 5, qui contient
- de 0,5 à 60 % en poids, de préférence de 1,0 à 50 % en poids, et en particulier de 1,0 à 40 % en poids, d'au moins un composé de formule générale I,
- de 0,01 à 5,0 % en poids, de préférence de 0,1 à 5,0 % en poids, et en particulier de 0,1 à 3,0 % en poids, d'amorceur(s) de polymérisation par voie radicalaire,
- et en option, de 5 à 80 % en poids, de préférence de 10 à 70 % en poids, et en particulier de 10 à 60 % en poids, d'acrylate(s) et/ou méthacrylate(s) polyfonctionnel(s),
dans chaque cas par rapport au poids total du matériau dentaire.

7. Matériau dentaire conforme à la revendication 6, qui présente la composition suivante :
a) de 5 à 80 % en poids, de préférence de 10 à 70 % en poids, et en particulier de 10 à 60 % en poids, d'acrylate(s) et/ou méthacrylate(s) polyfonctionnel(s),
b) de 0,01 à 5,0 % en poids, de préférence de 0,1 à 5,0 % en poids, et en particulier de 0,1 à 3,0 % en poids, d'amorceur(s),
c) de 0,5 à 60 % en poids, de préférence de 1,0 à 50 % en poids, et en particulier de 1,0 à 40 % en poids, d'au moins un composé de formule générale I,
d) de 0 à 50 % en poids, de préférence de 0 à 40 % en poids, et en particulier de 0 à 30 % en poids, d'acrylate(s) et/ou méthacrylate(s) monofonctionnel(s),
e) de 0 à 90 % en poids, de préférence de 5 à 90 % en poids, et en particulier de 0 à 80 % en poids, de charge(s),
f) et de 0 à 5 % en poids, de préférence de 0 à 3 % en poids, et en particulier de 0,2 à 3 % en poids, d'adjuvant(s),
dans chaque cas par rapport au poids total du matériau dentaire.

8. Matériau dentaire conforme à l'une des revendications 1 à 7, qui ne contient pas de mercaptans volatils, de préférence, ne contient pas du tout de mercaptans, et mieux encore, ne contient pas non plus d'autres composés soufrés.

9. Matériau dentaire conforme à l'une des revendications 1 à 8, pour utilisation intra-orale pour la restauration de dents endommagées.

10. Matériau dentaire conforme à la revendication 9, pour utilisation en tant que ciment, composite d'obturation ou matériau de revêtement.

11. Utilisation d'un matériau dentaire conforme à l'une des revendications 1 à 8 en tant que matériau pour fabrication ou réparation extra-orale de restaurations dentaires.

12. Utilisation conforme à la revendication 11 pour la fabrication de prothèses inlays ou onlays, de couronnes ou de bridges.

13. Utilisation d'un composé de formule (I), dans laquelle les symboles ont les définitions données dans la revendication 1 ou 2, pour faire baisser la tension de rétraction lors de la polymérisation de matériaux dentaires.
